Europäisches Patentamt

(19) European Patent Office

Office européen des brevets

(11) EP 0 659 393 B1

(12) **FASCICULE DE BREVET EUROPEEN**

(45) Date de publication et mention
de la délivrance du brevet:
**03.03.1999 Bulletin 1999/09**

(51) Int. Cl.<sup>6</sup>: **A61K 7/09**, A61K 7/06,
A45D 7/00

(21) Numéro de dépôt: **94402513.9**

(22) Date de dépôt: **07.11.1994**

(54) **Procédé de déformation non permanente des fibres kératiniques humaines**

Verfahren zur nicht-dauerhaften Haarverformung

Process for non-permanent hair deformation

(84) Etats contractants désignés:
**AT BE CH DE ES FR GB IT LI NL SE**

(30) Priorité: **22.12.1993 FR 9315479**

(43) Date de publication de la demande:
**28.06.1995 Bulletin 1995/26**

(73) Titulaire: **L'OREAL**
**75008 Paris (FR)**

(72) Inventeur: **Sturla, Jean-Michel**
**F-92210 Saint-Cloud (FR)**

(74) Mandataire:
**Tezier Herman, Béatrice**
**L'OREAL,**
**Département Propriété Industrielle,**
**90, rue du Gal Roguet**
**92583 Clichy Cédex (FR)**

(56) Documents cités:
**EP-A- 0 201 622          EP-A- 0 201 779**
**FR-A- 2 273 492**

## Description

La présente invention concerne un procédé amélioré de traitement des fibres kératiniques humaines, en particulier des cheveux, en vue notamment d'obtenir une déformation et/ou une mise en forme non permanente de ces dernières, en particulier sous la forme d'une mise en plis, ledit procédé étant notamment utilisable dans le domaine des salons de coiffure, de beauté, de cosmétique et analogues, professionnels. Plus précisément encore, elle concerne un procédé mettant en oeuvre de la vapeur d'eau et des substances traitantes particulières.

On sait que l'on désigne en coiffure sous le terme de "mise en plis" l'opération simple qui consiste à donner aux cheveux une forme non définitive et temporaire (généralement ondulée telle que boucles, frisures et autres) qui disparait instantanément lorsque les cheveux sont à nouveau mouillés, en particulier lorsque ceux-ci sont soumis à l'action de lavages à l'eau ou par shampooings, et ceci par opposition à une opération de déformation dite permanente, au cours de laquelle de véritables traitements et/ou transformations chimiques (réduction/oxydation) doivent être mis en oeuvre sur les fibres kératiniques, la forme finale imposée aux cheveux ne devenant dans ce cas plus du tout (ou que très peu) sensible aux agents extérieurs susmentionnées.

La technique la plus usuelle pour réaliser une mise en plis (ou déformation non permanente) des cheveux consiste à mettre tout d'abord sous tension (avec des supports classiques de type bigoudis, rouleaux de mise en plis et autres) des cheveux préalablement mouillés ou encore humides, puis à sécher les cheveux ainsi mis sous tension sous un casque de coiffure chauffant à une température comprise entre 30°C et 60°C pendant un temps qui peut varier de 20 à 60 mn selon la masse des cheveux à sécher, à ensuite retirer des cheveux ainsi séchés les moyens de mises sous tension utilisés précédemment et enfin à donner un coup de peigne de finition pour obtenir la coiffure avec la forme finale recherchée. Un autre procédé, moins usité, consiste à utiliser la technique ancienne dite du fer à friser ou du fer à coiffer (mèche de cheveux humide enroulée autour d'un mandrin métallique et portée par celui-ci à plus de 100°C pendant au moins 20 secondes); cette dernière technique est aujourd'hui peu pratiquée chez les coiffeurs professionnels car elle procure des résultats jugés dans l'ensemble peu satisfaisants et irréguliers en raison du fait, notamment, que les cheveux sont soumis à des températures très différentes selon qu'ils sont au contact même ou au contraire assez éloignés du mandrin chauffant.

Dans le document FR-A- 2 273 492, on a déja proposé de faire appel à des traitements à la vapeur d'eau surchauffée dans le but, entre autres, d'améliorer la qualité et/ou les rendements des mises en plis sur cheveux. Bien que cette technique permette effectivement, par rapport aux procédés classiques, d'améliorer tout de suite certaines caractéristiques de la mise en plis, et en particulier le rendement de la mise en plis (ou degré de frisure), ces améliorations restent néanmoins d'une durabilité (ou tenue) dans le temps limitée, puisque disparaissant généralement dans les quelques jours qui suivent le traitement. Par ailleurs, indépendamment de l'aspect rétention du degré de frisure évoqué précédemment, il serait bien évidemment intéressant de pouvoir disposer de cheveux bouclés présentant un degré de frisure initial (c.à.d. juste après traitement de mise en plis) encore amélioré par rapport à ce qui est connu à ce jour, avec ou sans traitement à la vapeur d'eau. Enfin, le procédé à la vapeur ci-dessus présente un autre inconvénient important, à savoir celui de conduire finalement à des fibres qui apparaissent rèches au toucher, ce qui n'est pas désirable d'un point de vue cosmétique.

La présente invention a notamment pour but de résoudre les problèmes ci-dessus.

Plus précisément encore, la présente invention a pour but de proposer un procédé de traitement convenant à la déformation non permanente des fibres kératiniques humaines, en particulier des cheveux, qui permette notamment d'obtenir des frisures de haute qualité.

Elle a également pour but de proposer un procédé tel que ci-dessus qui permette en outre de conserver ces frisures de manière durable dans le temps (rétention).

Elle a aussi pour but de proposer un procédé tel que ci-dessus permettant d'aboutir à des fibres kératiniques humaines déformées de manière non permanente tout en présentant des propriétés cosmétiques de douceur et de lissage remarquables.

Or, à la suite d'importantes recherches menées sur la question, il a maintenant été trouvé par la Demanderesse, et ceci de façon tout à fait inattendue et surprenante, que ces buts, et d'autres, pouvaient être atteints en mettant en oeuvre, selon certaines conditions particulières, de la vapeur d'eau sur des fibres kératiniques humaines préalablement traitées avec des silicones. Cette découverte est à la base de la présente invention.

Ainsi, il est maintenant proposé selon la présente invention un procédé de traitement convenant à la déformation et/ou la mise en forme, et ceci de manière non permanente, des fibres kératiniques humaines, et en particulier des cheveux, ledit procédé étant caractérisé par le fait qu'il consiste (i) à mettre en contact, pendant une durée n'excédant pas 2 minutes, un gaz contenant de la vapeur d'eau et dont la température est d'au moins 75°C, avec des fibres kératiniques humaines maintenues sous tension mécanique (rouleaux ou bigoudis par ex.) et sur lesquelles on a appliqué une composition contenant au moins une silicone, (ii) à refroidir les fibres ainsi traitées et enfin (iii) à supprimer la tension mécanique qui était appliquée sur les fibres.

Des modes de réalisation particuliers de la présent invention sont indiqués dans les revendications 2 à 14.

Bien que l'exposé qui suit s'articule essentiellement autour du cas particulier du traitement des cheveux, on notera ici que le procédé selon l'invention est applicable à toute matière kératinique humaine en général, notamment cils, moustaches, poils et autres.

Le gaz chaud traitant contient de préférence au moins 1% en volume de vapeur d'eau. En plus de la vapeur d'eau, le gaz vecteur (ou support) peut contenir de la vapeur de solvant, ainsi que des gaz tels que l'oxygène ou l'azote, des mélanges de gaz tels que l'air ou bien encore d'autres composés vaporisables.

A titre de solvants qui peuvent être avantageusement utilisés pour la production de vapeur (mélanges eau-solvant), on fait plus particulièrement appel aux solvants organiques cosmétiquement acceptables, comme par exemple des alcools tels que l'éthanol, l'isopropanol, l'alcool benzylique, l'alcool phényléthylique ou des glycols ou éthers de glycol tels que par exemple l'éthylène glycol et ses éthers monométhylique, monoéthylique et monobutylique, le propylène glycol, le butylène glycol, le dipropylèneglycol, ainsi que les alkyléthers comme le monobutyléther du diéthylèneglycol.

Selon la présente invention, le gaz est constitué de préférence soit uniquement ou essentiellement de vapeur d'eau, soit d'un mélange d'eau et d'air.

La température du gaz est de préférence supérieure ou égale à 85°C, et est plus particulièrement comprise entre 85°C et 150°C.

Selon une caractéristique importante du procédé selon l'invention, le temps de contact entre le gaz chaud traitant et la fibre doit être bref, et en tout cas ne pas excéder 2 minutes. De préférence, le gaz est mis en contact avec la fibre pendant une durée allant de 0,01 seconde à 30 secondes, et encore plus préférentiellement de 1 seconde à 10 secondes. Bien entendu, l'application du gaz peut être répétée plusieurs fois sur la même fibre, chaque opération se faisant selon une durée telle qu'indiquée ci-dessus.

Un mode préféré de réalisation du procédé selon l'invention consiste à appliquer tout d'abord sur les cheveux une composition constituée pour partie ou totalement de silicones, cette application pouvant être réalisée avant, pendant ou après l'habituelle opération de mise sous tension des mèches de cheveux sous une forme correspondant à la forme finale désirée pour ces derniers (boucles par exemple), cette opération pouvant elle-même être mise en oeuvre par tout moyen, mécanique notamment, approprié et connu en soi pour maintenir sous tension des cheveux, tels que par exemple corps tubulaires, rouleaux, bigoudis et autres, puis à soumettre ces mèches ainsi imprégnées de silicones à l'action brève de la vapeur d'eau selon les conditions mentionnées ci-avant, puis à refroidir, de préférence rapidement, les mèches ainsi traitées à la vapeur, par exemple en envoyant sur ou à travers celles-ci un courant d'air à température ambiante et/ou en aspirant un courant d'air ambiant à travers les mèches enroulées, et enfin à retirer des cheveux les moyens mécaniques qui maintenaient ces derniers sous tension et dans la forme désirée tout au long du traitement, ce par quoi l'on obtient finalement des mèches ou une chevelure présentant par exemple de belles boucles régulières et douces.

La production d'un gaz chaud comprenant de la vapeur d'eau peut se faire à l'aide de tout appareil connu en soi et prévu à cet effet. Toutefois, selon la présente invention, on utilise de préférence un appareil tel que celui décrit dans la demande de brevet français FR-A- 2 273 492, ou tout autre appareil équivalent, qui convient en effet dans le cas présent particulièrement bien (traitement ponctuel, régulier et homogène des fibres, sans risque de surchauffe, avec post-traitement de refroidissement intégré).

Selon la présente invention, il est possible d'utiliser toute silicone connue en soi, qu'il s'agisse d'une huile, d'une résine ou bien encore d'un élastomère (gomme) silicone. Les silicones sont des polymères ou oligomères organosiliciés à structure linéaire ou cyclique, ramifiée ou réticulée, de poids moléculaire variable, obtenus par polymérisation et/ou polycondensation de silanes convenablement fonctionalisés, et constitués pour l'essentiel par une répétition de motifs principaux dans lesquels les atomes de silicium sont reliés entre eux par des atomes d'oxygène (liaison siloxane), des radicaux hydrocarbonés éventuellement substitués étant directement liés par l'intermédiaire d'un atome de carbone sur lesdits atomes de silicicium. Les radicaux hydrocarbonés les plus courants sont les radicaux alkyles et en particulier méthyle, les radicaux fluoroalkyles, les radicaux aryles et en particulier phényle, et les radicaux alcényles et en particulier vinyle; d'autres types de radicaux susceptibles d'être liés soit directement, soit par l'intermédiaire d'un radical hydrocarboné, à la chaine siloxanique sont notamment l'hydrogène, les halogènes et en particulier le chlore, le brome ou le fluor, les thiols, les radicaux alcoxys, les radicaux polyoxyalkylènes (ou polyéthers) et en particulier polyoxyéthylène et/ou polyoxypropylène, les radicaux hydroxyles ou hydroxyalkyles, les groupements aminés substitués ou non, les groupements amides, les radicaux acyloxys ou acyloxyalkyles, les radicaux hydroxyalcylamino ou aminoalkyles, des groupements ammonium quaternaires, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites "organomodifiées"). D'une manière générale, les silicones utilisables dans le cadre de la présente invention sont celles qui sont notamment décrites dans "Encyclopedia of Chemical Technology, Kirk-Othmer, Third Edition, 1982, volume 20, pp. 922 et suivantes" et dans "Chemistry and Technology of Silicones, Walter NOLL, Academic Press Inc, San Diego California, 1968". Il est également possible d'utiliser des copolymères blocs linéaires comprenant dans leur chaine principale des segment polysiloxaniques, comme par exemple des copolymères blocs polysiloxane-polyoxyalkylène ou bien encore polysiloxane-polyuréthane et/ou polyurée. Le poids moléculaire moyen des silicones utilisables selon l'invention peut varier entre 100 et plusieurs millions, de préférence entre 1000 et 1 000 000. Selon la présente invention, on peut bien entendu soit utiliser une seule et même silicone soit, au contraire, mettre en oeuvre plusieurs silicones différentes.

A titre d'exemples de silicones convenant à la mise en oeuvre du procédé selon l'invention, on peut notamment citer les silicones volatiles, les huiles de silicone linéaires ou cycliques et leurs mélanges, les polydiméthylsiloxanes, les polyorganosiloxanes quaternisés tels que ceux décrits dans la demande de brevet français n° 2 535 730, les polyorganosiloxanes à groupements aminoalkyles modifiés par des groupements alcoxycarbonylalkyles tels que ceux décrits dans le brevet US n° 4 749 732, des polyorganosiloxanes tels que le copolymère polydiméthylsiloxane-polyoxyalkyle du type Diméthicone Copolyol, un polydiméthylsiloxane à groupements terminaux stéaroxy- (stéaroxydiméthicone), un copolymère polydiméthylsiloxane-dialkylammonium acétate ou un copolymère polydiméthyl-siloxane polyalkylbétaïne décrits dans la demande de brevet britannique n° 2 197 352, des polysiloxanes organo modifiés par des groupements mercapto ou mercaptoalkyles tels que ceux décrits dans le brevet français n° 1 530 369 et dans la demande de brevet européen n° 295 780, et d'une manière encore plus générale tous les organopolysiloxanes décrits dans la demande de brevet publiée sous le numéro WO 93/05762 .

Selon un mode particulièrement préféré de mise en oeuvre du procédé selon l'invention, les silicones utilisées sont choisies parmi les diorganopolysiloxanes (huiles, gommes ou résines), de préférence les dialkylpolysiloxanes ou les alkylarylpolysiloxane, et encore plus préférentiellement les diméthylpolysiloxanes, toutes ces silicones pouvant éventuellement contenir des radicaux polyoxyalkylènes, de préférence polyoxyéthylène et/ou polyoxypropylène, greffés sur certains atomes de silicium (polymères organomodifiés de type Diméthieone Copolyol selon la dénomination CTFA).

Selon la présente invention, on peut mettre en oeuvre les silicones seules, mais le plus souvent on fait appel à des compositions contenant, dans un support cosmétiquement acceptable, de telles silicones. Elles peuvent ainsi être soit solubilisées, soit mises en dispersions ou en (micro)émulsions, dans des milieux aqueux ou des milieux organiques ou encore des milieux hydroorganiques. A titre de solvants organiques convenables, on peut notamment citer les monoalcools ou les polyols (éthanol, isopropanol, glycérol, alcool benzylique, glycols), l'acétone, les éthers de polyols, les hydrocarbures, les esters ou bien encore les silicones volatiles.

Les teneurs en silicone(s) dans les compositions peuvent varier dans de très larges limites et être par exemple comprises entre 0,01 % et 50 %, de préférence entre 0,1 et 10%, en poids par rapport à l'ensemble de la composition.

Les compositions peuvent se présenter sous toute forme habituellement utilisée dans le domaine des compositions capillaires à usage topique, telle que par exemple liquide plus ou moins épaissi ou gélifié, crème, mousse, lotion, gel, pâte, émulsion, aérosol ou toute autre forme appropriée.

Les compositions à base de silicone peuvent ainsi, et d'une manière générale, contenir tous les divers additifs classiques qui sont utilisés dans le domaine de la préparation des compositions capillaires à usage topique et être choisis par exemple parmi des filtres UV, des agents épaississants, des agents de pénétration, des anti-oxydants, des agents sequestrants, des agents opacifiants, des tampons, des agents tensio-actifs choisis parmi les tensio-actifs non-ioniques tels que les alkylpolyglycosides, les tensio-actifs cationiques, les tensio-actifs anioniques et les tensio-actifs amphotères, des agents solubilisants, des émollients, des colorants, des parfums et des conservateurs.

Les compositions à base de silicone utilisées dans le cadre de la présente invention, qui sont donc destinées à être appliquées sur des cheveux, présentent de préférence un pH compris entre 3 et 11; si nécessaire, ce pH peut être ajusté à la valeur désirée par ajout, selon les cas, soit d'agents basifiants, soit d'agents acidifiants, usuels et connus comme cosmétiquement acceptables.

Des exemples concrets, mais nullement limitatifs, illustrant l'invention vont maintenant être donnés. Aux fins d'une comparaison significative, les mêmes mèches de cheveux de départ (avant traitement) ont été utilisées pour tous les exemples.

## EXEMPLE 1

On a utilisé une composition 1 à base de silicone présentant les caractéristiques suivantes (% en poids) :

| | |
|---|---|
| - Huile silicone commercialisée par RHONE-POULENC sous le nom de marque SILBIONE HUILE 70 646 (polydiméthylsiloxane oxyéthyléné et oxypropyléné; 100% MA; dénomination CTFA : Diméthicone Copolyol) | 1 % |
| - Ethanol      qsp | 100 % |

Cette composition est conditionnée dans un flacon-pompe libérant des doses de 0,15 ml par pulvérisation.

Le mode opératoire est le suivant : on applique sur une mèche de cheveux naturels 4 pulvérisations de la composition ci-dessus; puis on enroule la mèche ainsi traitée sur un bigoudis dont le diamètre est de 20 mm; puis on traite pendant 5 secondes la mèche ainsi enroulée au moyen d'un jet de gaz ne contenant pour l'essentiel que de la vapeur d'eau et dont la température est de 85°C; la mèche ainsi traitée est ensuite rapidement refroidie au moyen d'un courant d'air ambiant, enfin on déroule la mèche du bigoudis.

Pour quantifier l'efficacité du procédé selon l'invention, on a d'abord mesuré la longueur initiale $L_0$ (en cm) de la mèche de cheveux avant le traitement à la vapeur (longueur mesurée entre les racines et les pointes sur mèche suspendue verticalement); de la même manière, on a mesuré ensuite la longueur $L_1$ de cette même mèche juste en fin de traitement; et enfin, on a mesuré la longueur $L_2$ de cette mèche 48 heures après le traitement.

On définit le rendement (en %) de la mise en plis par le rapport

$$\rho = \frac{L_0 - L_1}{L_0} \times 100$$

On définit la rétention (en %) de la mise en plis par le rapport

$$r = \frac{L_0 - L_2}{L_0} \times 100$$

La mise en plis sera d'autant meilleure que l'un et/ou l'autre (et de préférence les deux à la fois) de ces deux rapports sera élevé.

Les résultats obtenus ont été les suivants :

$L_0 = 24cm$; $\qquad L_1 = 16cm\ (\rho = 33\%)$; $\qquad L_2 = 19cm\ (r = 21\%)$

A titre comparatif, les résultats obtenus dans le cas d'un traitement à la vapeur conduit sur des mèches non préalablement traitées par des silicones, ont été les suivants :

$L_0 = 24cm$; $\qquad L_1 = 18cm\ (p = 25\%)$; $\qquad L_2 = 23cm\ (r = 4\%)$

## EXEMPLE 2

On procède comme à l'exemple 1, à cette différence près qu'une composition 2 à base de silicone ayant les caractéristiques suivantes (% en poids) a été utilisée :

| | |
|---|---|
| - Huile de silicone commercialisée par GOLDSCHMIDT sous le nom de marque ABIL B 8842 (polydiméthyl/méthylsiloxane oxyéthyléné (18/5); 100% MA; viscosité : 500 mm$^2$.s$^{-1}$ (cSt) environ; poids moléculaire : 6000 environ; dénomination CTFA : Diméthicone Copolyol) | 0,8 % |
| - Eau | 10 % |
| - Ethanol          qsp | 100 % |

Les résultats obtenus sont comparables à ceux de l'exemple 1.

## EXEMPLE 3

On procède comme à l'exemple 1, à cette différence près qu'une composition 3 à base de silicone ayant les caractéristiques suivantes (% en poids) a été utilisée :

| | |
|---|---|
| - Emulsion de silicone commercialisée par DOW CORNING sous le nom de marque EMULSION CATIONIQUE DC 929 (polydiméthylsiloxane à groupements aminoéthylpropylamine et $\alpha,\omega$ disilanols en émul-sion aqueuse cationique; 35% en MA; dénomination CTFA : Amodiméthicone) | 10 % |
| - Eau     qsp | 100 % |

Les résultats obtenus sont comparables à ceux de l'exemple 1.

EXEMPLE 4

On procède comme à l'exemple 1, à cette différence près qu'une composition 4 à base de silicone ayant les caractéristiques suivantes (% en poids) a été utilisée :

| | |
|---|---|
| - Huile de silicone commercialisée par GOLDSCHMIDT sous le nom de marque ABIL AV 1000 (polyméthylphénylsiloxane; 100% en MA; dénomination CTFA : Phényl Triméthicone) | 1 % |
| - Silicone volatile       qsp | 100 % |

Les résultats obtenus sont comparables à ceux de l'exemple 1.

EXEMPLE 5

On procède comme à l'exemple 1, à cette différence près qu'une composition 5 à base de silicone ayant les caractéristiques suivantes (% en poids) a été utilisée :

| | |
|---|---|
| - Polydiméthylsiloxane en microémulsion aqueuse anionique; 16 % en MA; viscosité : 100 $mm^2.s^{-5}$ (cSt) environ; $\Phi_{particules}$ : 50 nm; dénomination CTFA : Diméthicone) | 5 % |
| -Eau       qsp | 100 % |

Les résultats obtenus sont comparables à ceux de l'exemple 1.
Tous les résultats ci-dessus démontrent clairement les améliorations apportées au niveau du rendement et de la rétention pour les mises en plis obtenues par le procédé selon l'invention.
Par ailleurs, toutes les mèches de cheveux pré-traitées par une silicone présentaient finalement au toucher une douceur et un lissage remarquables.

**Revendications**

1.  Procédé de traitement pour la déformation non permanente des fibres kératiniques humaines, en particulier des cheveux, caractérisé par le fait qu'il consiste (i) à mettre en contact, pendant une durée n'excédant pas 2 minutes, un gaz contenant de la vapeur d'eau et dont la température est d'au moins 75°C, avec des fibres kératiniques humaines maintenues sous tension mécanique et sur lesquelles on a appliqué une composition contenant au moins une silicone, (ii) à refroidir les fibres ainsi traitées et enfin (iii) à supprimer la tension mécanique qui était appliquée sur les fibres.

2.  Procédé selon la revendication 1, caractérisé par le fait que ledit gaz a une température supérieure ou égale à 85°C.

3.  Procédé selon la revendication 2, caractérisé par le fait que ladite température est comprise entre 85°C et 150°C.

4.  Procédé selon l'une quelconque des revendications précédentes, caractérisé par le fait que ledit gaz est mis en contact avec la fibre à déformer pendant une durée allant de 0,01 seconde à 2 minutes.

**5.** Procédé selon la revendication 4, caractérisé par le fait que ladite durée est comprise entre 0,01 seconde et 30 secondes.

**6.** Procédé selon la revendication 5, caractérisé par le fait que ladite durée est comprise entre 1 seconde et 10 secondes.

**7.** Procédé selon l'une quelconque des revendications précédentes, caractérisé par le fait que l'application du gaz est répétée plusieurs fois sur une même fibre.

**8.** Procédé selon l'une quelconque des revendications précédentes, caractérisé par le fait que ledit gaz ne contient que de la vapeur d'eau.

**9.** Procédé selon l'une quelconque des revendications 1 à 7, caractérisé par le fait que ledit gaz contient de la vapeur d'eau et au moins un autre composé sous forme de gaz ou de vapeur.

**10.** Procédé selon la revendication 9, caractérisé par le fait que ledit gaz contient de la vapeur d'eau et de l'air.

**11.** Procédé selon l'une quelconque des revendications précédentes, caractérisé par le fait que la silicone est choisie parmi les silicones volatiles, les huiles silicones, les résines silicones ou les gommes silicones.

**12.** Procédé selon la revendication 11, caractérisé par le fait que la silicone est choisie parmi les diorganopolysiloxanes éventuellement modifiés.

**13.** Procédé selon la revendication 12, caractérisé par le fait que la silicone est choisie parmi les dialkylpolysiloxanes et les alkylarylpolysiloxanes, éventuellement modifiés.

**14.** Procédé selon la revendication 13, caractérisé par le fait que la silicone est choisie parmi les diméthylpolysiloxanes, éventuellement modifiés.

**Claims**

**1.** Treatment process for the non-permanent reshaping of human keratinous fibres, especially hair, characterized in that it consists (i) in bringing a gas containing water vapour, and the temperature of which is at least 75°C, into contact for a time not exceeding 2 minutes with human keratinous fibres maintained under mechanical tension and to which a composition containing at least one silicone has been applied, (ii) in cooling the fibres thus treated, and lastly (iii) in removing the mechanical tension which was applied to the fibres.

**2.** Process according to Claim 1, characterized in that the said gas has a temperature above or equal to 85°C.

**3.** Process according to Claim 2, characterized in that the said temperature is between 85°C and 150°C.

**4.** Process according to any one of the preceding claims, characterized in that the said gas is brought into contact with the fibre to be reshaped for a time ranging from 0.01 second to 2 minutes.

**5.** Process according to Claim 4, characterized in that the said time is between 0.01 second and 30 seconds.

**6.** Process according to Claim 5, characterized in that the said time is between 1 second and 10 seconds.

**7.** Process according to any one of the preceding claims, characterized in that the application of the gas is repeated several times on the same fibre.

**8.** Process according to any one of the preceding claims, characterized in that said gas contains only water vapour.

**9.** Process according to any one of Claims 1 to 7, characterized in that the said gas contains water vapour and at least one other compound in gas or vapour form.

**10.** Process according to Claim 9, characterized in that the said gas contains water vapour and air.

11. Process according to any one of the preceding claims, characterized in that the silicone is chosen from volatile silicones, silicone oils, silicone resins and silicone gums.

12. Process according to Claim 11, characterized in that silicone is chosen from diorganopolysiloxanes, optionally modified.

13. Process according to Claim 12, characterized in that the silicone is chosen from dialkylpolysiloxanes and alkylarylpolysiloxanes, optionally modified.

14. Process according to Claim 13, characterized in that the silicone is chosen from dimethylpolysiloxanes, optionally modified.

**Patentansprüche**

1. Verfahren zur Behandlung von menschlichen Keratinfasern und insbesondere zur Behandlung des Haares zur nicht dauerhaften Verformung, dadurch gekennzeichnet, daß es aus folgenden Schritten besteht:

   (i) Inkontaktbringen eines Gases, das Wasserdampf enthält und dessen Temperatur mindestens 75 °C beträgt, mit menschlichen Keratinfasern, die unter mechanischer Spannung gehalten werden und auf die eine Zusammensetzung aufgebracht wurde, die mindestens ein Silicon enthält, während einer Zeitspanne, die 2 min nicht übersteigt,
   (ii) Abkühlen der so behandelten Fasern, und schließlich
   (iii) Aufheben der mechanischen Spannung, die auf die Fasern ausgeübt worden ist.

2. Verfahren nach Anspruch 1, dadurch gekennzeichnet, daß das Gas eine Temperatur von mindestens 85 °C aufweist.

3. Verfahren nach Anspruch 2, dadurch gekennzeichnet, daß die Temperatur im Bereich von 85 bis 150 °C liegt.

4. Verfahren nach einem der vorhergehenden Ansprüche, dadurch gekennzeichnet, daß das Gas mit den zu verformenden Fasern während einer Dauer von 0,01 s bis 2 min in Kontakt gebracht wird.

5. Verfahren nach Anspruch 4, dadurch gekennzeichnet, daß die Dauer 0,01 s bis 30 s beträgt.

6. Verfahren nach Anspruch 5, dadurch gekennzeichnet, daß die Dauer 1 s bis 10 s beträgt.

7. Verfahren nach einem der vorhergehenden Ansprüche, dadurch gekennzeichnet, daß man das Gas mehrmals auf die gleichen Fasern einwirken läßt.

8. Verfahren nach einem der vorhergehenden Ansprüche, dadurch gekennzeichnet, daß das Gas ausschließlich Wasserdampf enthält.

9. Verfahren nach einem der Ansprüche 1 bis 7, dadurch gekennzeichnet, daß das Gas Wasserdampf und mindestens eine weitere Verbindung in Form von Gas oder Dampf enthält.

10. Verfahren nach Anspruch 9, dadurch gekennzeichnet, daß das Gas Wasserdampf und Luft enthält.

11. Verfahren nach einem der vorhergehenden Ansprüche, dadurch gekennzeichnet, das das Silicon unter den flüchtigen Siliconen, den Siliconölen, den Siliconharzen und den Silicongummis ausgewählt ist.

12. Verfahren nach Anspruch 11, dadurch gekennzeichnet, daß das Silicon unter den Diorganopolysiloxanen ausgewählt ist, die gegebenenfalls modifiziert sind.

13. Verfahren nach Anspruch 12, dadurch gekennzeichnet, daß das Silicon unter den Dialkylpolysiloxanen und den Alkylarylpolysiloxanen ausgewählt ist, die gegebenenfalls modifiziert sind.

14. Verfahren nach Anspruch 13, dadurch gekennzeichnet, daß das Silicon unter den Dimethylpolysiloxanen ausgewählt ist, die gegebenenfalls modifiziert sind.